Europäisches Patentamt

European Patent Office

Office européen des brevets

(11) Publication number: **0 344 767**
**A1**

# EUROPEAN PATENT APPLICATION

(21) Application number: **89109923.6**

(51) Int. Cl.⁴: **C12N 15/00**

(22) Date of filing: **01.06.89**

The applicant has filed a statement in accordance with Rule 28 (4) EPC (issue of a sample only to an expert). Accession number(s) of the deposit(s): FERM BP-1613, BP-1614, BP-1615, BP-1616, BP-1617, BP-1618 and BP-1770.

(30) Priority: **02.06.88 JP 136225/88**

(43) Date of publication of application:
**06.12.89 Bulletin 89/49**

(84) Designated Contracting States:
**AT BE CH DE ES FR GB GR IT LI LU NL SE**

(71) Applicant: **KYOWA HAKKO KOGYO CO., LTD.**
**6-1, Ohte-Machi Itchome**
**Chiyoda-ku Tokyo(JP)**

(72) Inventor: **Hasegawa, Mamoru**
**1-9-26, Katahira Asao-ku**
**Kawasaki-shi Kanagawa-ken(JP)**
Inventor: **Dairi, Toru**
**4-17-9-102, Morino**
**Machida-shi Tokyo(JP)**
Inventor: **Kawamoto, Isao**
**1-21-2, Fujimino**
**Hiratsuka-shi Kanagawa-ken(JP)**

(74) Representative: **Vossius & Partner**
**Siebertstrasse 4 P.O. Box 86 07 67**
**D-8000 München 86(DE)**

(54) Cloning vectors.

(57) According to the present invention, a cloning vector capable of autonomous replication of a microorganism of the genus Micromonospora can be obtained and a host-vector system for a microorganism of the genus Micromonospora can be provided.

EP 0 344 767 A1

## CLONING VECTORS

The present invention relates to cloning vectors capable of autonomous replication in a microorganism of the genus Micromonospora. Thus, the present invention provides vectors useful in application of recombinant DNA technology to a microorganism of the genus Micromonospora.

Heretofore, development of the recombinant DNA technology on actinomycetes has been made mainly on microorganisms of the genus Streptomyces [K.F. Chater, et al.: Current Topics in Microbiology and Immunology, 96, 69-95 (1982)].

Vectors derived from microorganisms of the genus Streptomyces and so far studied have not been applicable to gene cloning in actinomycetes other than those of the genus Streptomyces, because they are not autonomously replicated or are not stably maintained in these actinomycetes [J. Antibiotics, 41 No. 4, 583-585 (1988)]. Actinomycetes other than microorganisms of the genus Streptomyces, particularly microorganisms of the genus Micromonospora, are known as microorganisms producing such antibiotics as fortimicin, sagamicin and gentamicins. Clarification of biosynthetic systems of these substances is acquiring great importance for producing these substances. However, as there have not been developed vectors capable of autonomous replication in a microorganism of the genus Micromonospora and having a transformation efficiency equivalent to that of the genetic recombination system for the genus Streptomyces, the recombinant DNA technology has not been applied to microorganisms of the genus Micromonospora yet. Thus development of host-vector systems for microorganisms of the genus Micromonospora has been desied.

In order to develop the host-vector systems for microorganisms of the genus Micromonospora, the present inventors have made extensive studies of plasmids capable of autonomous replication in microorganisms of the genus Micromonospora. As the result, plasmids capable of autonomous replication in microorganisms of the genus Micromonospora have been found and isolated from Micromonospora olivasterospora ATCC 31010 and Micromonospora olivasterospora 3629-4 (FERM BP-1614). Furthermore, the present inventors have developed cloning vectors for microorganisms of the genus Micromonospora by utilizing the capability of these plasmids to autonomously replicate in these microorganisms and incorporating appropriate vector-markers into the plasmids.

The present invention provides plasmids capable of autonomous replication in a microorganism of the genus Micromonospora or both of a microorganism of the genus Micromonospora and a microorganism of Escherichia coli.

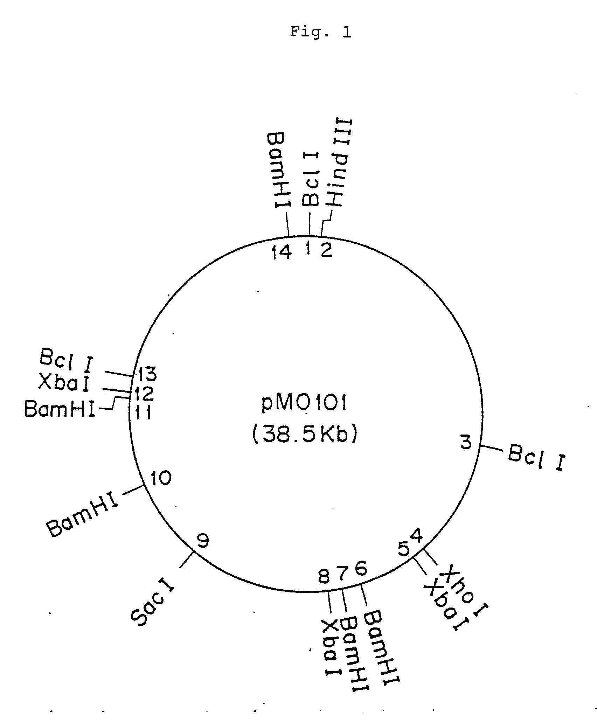

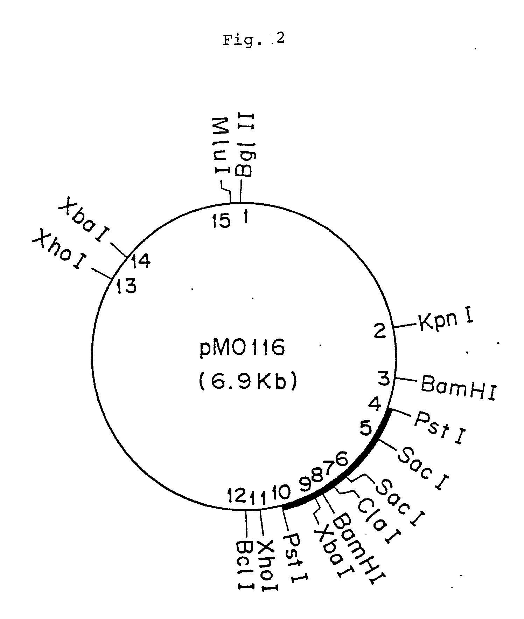

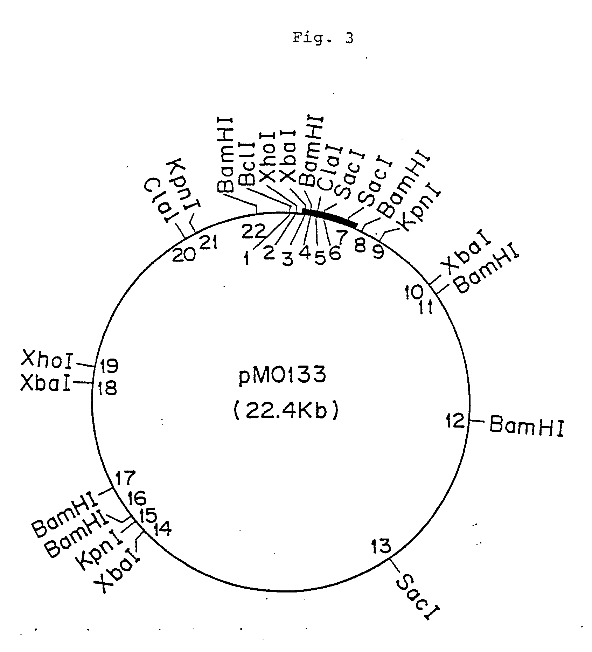

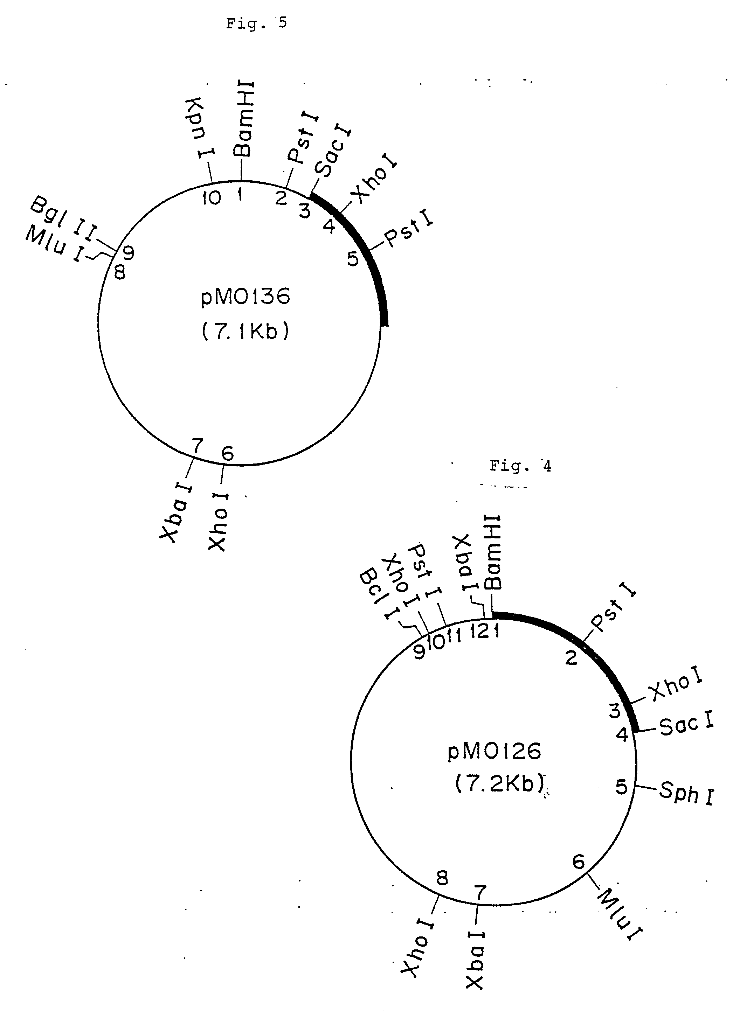

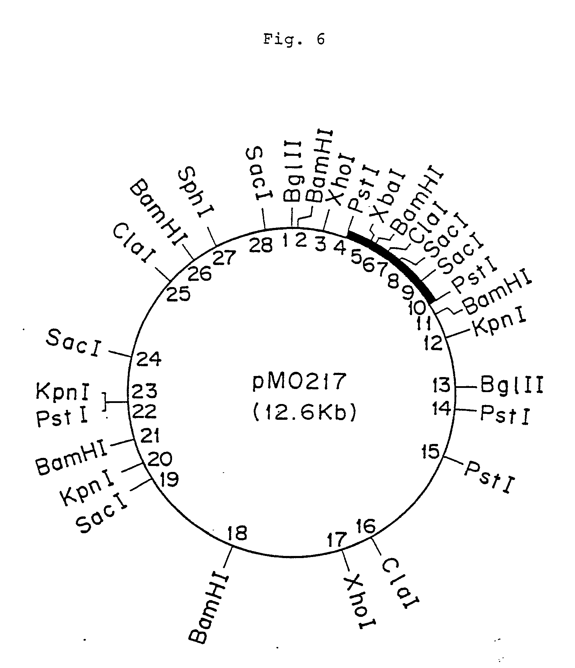

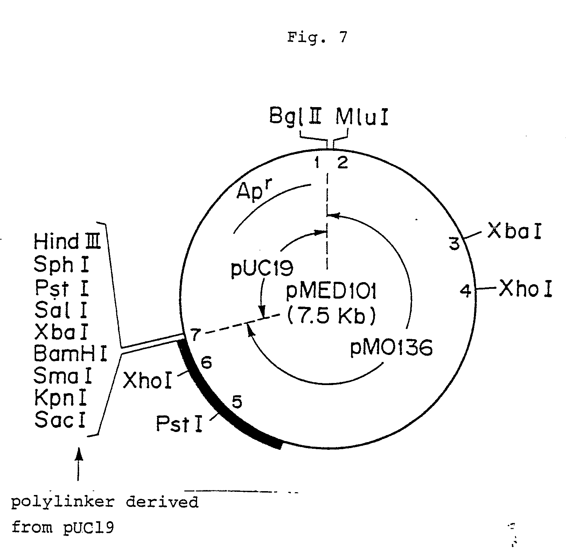

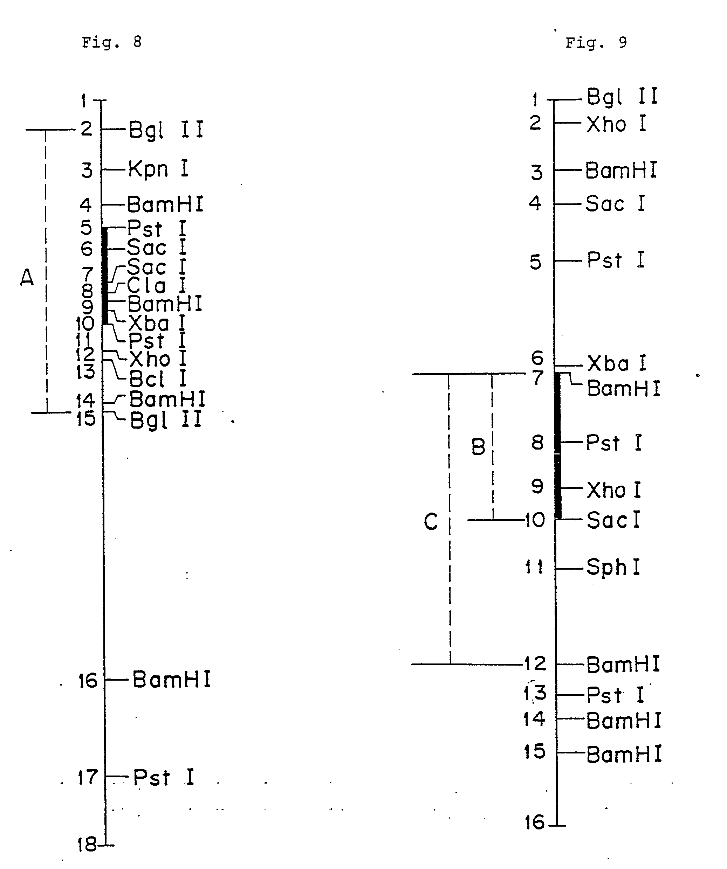

Figs. 1 to 7 are restriction enzyme cleavage maps for pMO101, pMO116, pMO133, pMO126, pMO136, pMO217 and pMED101, respectively. Figs. 8 and 9 are restriction enzyme cleavage maps for DNA fragments containing neomycin-resistance genes derived from Micromonospora sp. MK-50. In the drawings, the neomycin-resistance genes exist in the region indicated by a bold line.

Part A in Fig. 8 is the DNA fragment inserted into pMO116, pMO133 and pMO217. Part B in Fig. 9 is the DNA fragment inserted into pMO126 and Part C is the DNA fragment inserted into pMO136 and pMED101. The numbers in the drawings represent the following kb values, respectively.

```
Fig. 1:   1.  0.00/38.5    6. 17.3      11. 29.0

          2.  0.51         7. 17.9      12. 29.2
```

|  |  |  |  |  |  |  |
|---|---|---|---|---|---|---|
|  | 3. | 10.5 | 8. | 18.3 | 13. | 29.9 |
|  | 4. | 14.9 | 9. | 23.3 | 14. | 37.1 |
|  | 5. | 15.2 | 10. | 26.4 |  |  |
| Fig. 2: | 1. | 0.00/6.9 | 6. | 2.7 | 11. | 3.4 |
|  | 2. | 1.5 | 7. | 2.8 | 12. | 3.5 |
|  | 3. | 1.9 | 8. | 2.9 | 13. | 5.8 |
|  | 4. | 2.1 | 9. | 2.95 | 14. | 6.0 |
|  | 5. | 2.35 | 10. | 3.1 | 15. | 6.8 |
| Fig. 3: | 1. | 0.00/22.4 | 9. | 2.0 | 17. | 15.1 |
|  | 2. | 0.10 | 10. | 3.2 | 18. | 17.2 |
|  | 3. | 0.50 | 11. | 3.4 | 19. | 17.5 |
|  | 4. | 0.60 | 12. | 5.9 | 20. | 20.9 |
|  | 5. | 0.70 | 13. | 8.35 | 21. | 21.0 |
|  | 6. | 0.80 | 14. | 14.1 | 22. | 22.0 |
|  | 7. | 1.15 | 15. | 14.4 |  |  |
|  | 8. | 1.6 | 16. | 14.5 |  |  |
| Fig. 4: | 1. | 0.00/7.2 | 6. | 3.1 | 11. | 6.9 |
|  | 2. | 0.75 | 7. | 3.9 | 12. | 7.0 |
|  | 3. | 1.25 | 8. | 4.2 |  |  |
|  | 4. | 1.50 | 9. | 6.5 |  |  |
|  | 5. | 2.05 | 10. | 6.6 |  |  |
| Fig. 5: | 1. | 0.00/7.1 | 6. | 4.2 |  |  |
|  | 2. | 0.20 | 7. | 4.4 |  |  |
|  | 3. | 0.40 | 8. | 5.2 |  |  |
|  | 4. | 0.70 | 9. | 5.3 |  |  |
|  | 5. | 1.2 | 10. | 6.8 |  |  |
| Fig. 6: | 1. | 0.00/12.6 | 11. | 2.2 | 21. | 9.3 |
|  | 2. | 0.10 | 12. | 2.6 | 22. | 9.6 |
|  | 3. | 0.60 | 13. | 3.0 | 23. | 9.6 |
|  | 4. | 1.0 | 14. | 3.3 | 24. | 9.9 |
|  | 5. | 1.1 | 15. | 4.2 | 25. | 10.6 |
|  | 6. | 1.2 | 16. | 5.9 | 26. | 10.8 |
|  | 7. | 1.3 | 17. | 6.4 | 27. | 11.5 |
|  | 8. | 1.4 | 18. | 7.5 | 28. | 12.1 |
|  | 9. | 1.75 | 19. | 8.9 |  |  |

```
                    10.  2.0        20.  9.2
         Fig. 7:    1.  0.00/7.5     6.  4.6
                    2.  0.10         7.  4.9
                    3.  0.90
                    4.  0.93
                    5.  4.1
         Fig. 8:    1.  0.00         7.  1.92      13.  2.75
                    2.  0.30         8.  2.02      14.  3.20
                    3.  0.72         9.  2.12      15.  3.30
                    4.  1.10        10.  2.21      16.  6.10
                    5.  1.32        11.  2.35      17.  7.10
                    6.  1.57        12.  2.65      18.  7.80
         Fig. 9:    1.  0.00         7.  2.88      13.  6.25
                    2.  0.25         8.  3.61      14.  6.50
                    3.  0.76         9.  4.10      15.  6.86
                    4.  1.10        10.  4.42      16.  7.70
                    5.  1.69        11.  4.93
                    6.  2.80        12.  5.91
```

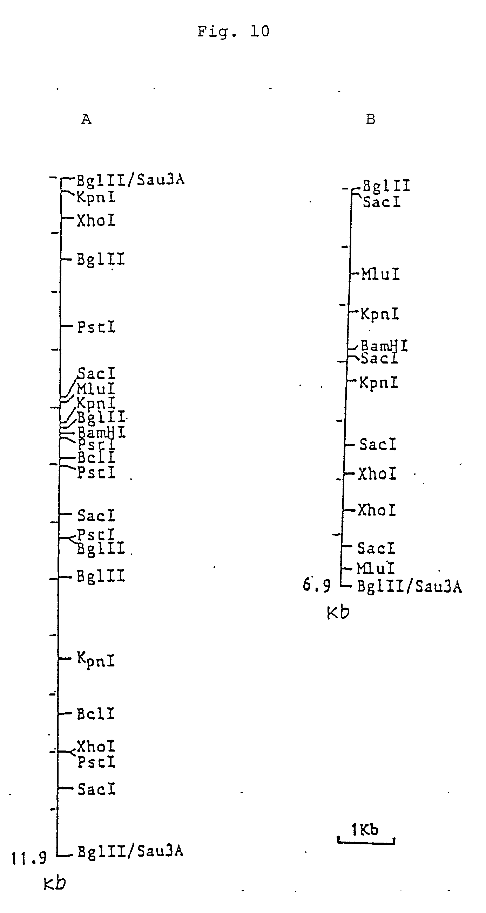

Fig. 10 A and B are restriction enzyme cleavage maps for pAN 25-10 and pAN 58-10, respectively.

As the specific plasmids capable of autonomous replication in a microorganism of the genus Micromonospora, plasmid pMO101 isolated from Micromonospora olivasterospora ATCC 31010, and plasmid pMO201 isolated from Micromonospora olivasterospora 3629-4 (FERM BP-1614) can be mentioned. These plasmids can be readily separated from cells containing them according to the procedure disclosed in Plasmid 12, 19 (1984).

Plasmid pMO101 is a circular DNA having a molecular weight of about 25 megadaltons. Numbers of sites sensitive to various restriction enzymes are shown in Table 1. pMO101 has no sites sensitive to Eco RI and Bgl II.

Table 1

| Cleavage Characteristics of pMO101 with Restriction Enzymes | | |
|---|---|---|
| Restriction enzyme | No. of cleavage sites | Length of DNA fragments formed by cleavage (kb) |
| Hind III | 1 | 38.5 |
| Bam HI | 5 | 1.0, 4.0, 7.8, 8.5, 18 |
| Bcl I | 3 | 8.5, 10.5, 19 |
| Pst I | 9 | 1.9, 2.2, 2.5, 2.9, 3.0, 3.6, 5.1, 6.8, 10.2 |
| Sac I | 1 | 38.5 |
| Xba I | 3 | 3.1, 11.0, 24 |
| Xho I | 1 | 38.5 |
| Kpn I | 6 | 1.4, 1.6, 3.1, 6.5, 11.5, 14 |

Plasmid pMO201 is revealed to be a plasmid of 40 kilobases (kb) or more by agarose gel electrophoresis and shows a distinct band below the band of chromosomal DNA by ethidium bromide-cesium chloride density-gradient centrifugation, and thus seems to have a closed circular DNA structure. However, its digestion with various restriction enzymes yields a large number of ladder-DNA bands by agarose gel electrophoresis. This fact suggests that pMO201 is a mixture of two or more plasmids of considerably large molecular size. Thus, the structure of pMO201 has not been determined. Nevertheless, various vectors can be constructed by utilizing the DNA parts of the plasmid which harbor the function of autonomous replication in a microorganism of the genus Micromonospora.

The above-mentioned plasmids have no vector-markers necessary for detecting them in microorganisms. In order to establish a host-vector system for a microorganism of the genus Micromonospora, a cloning vector having an appropriate vector-marker for detecting the vector is desirable. Such cloning vectors can be constructed by utilizing the ability of the above plasmids to autonomously replicate in a microorganism of the genus Micromonospora and incorporating an appropriate vector-marker into the plasmids.

As the vector-marker, any DNA fragment can be used so long as it contains a gene which can be expressed in a microorganism of the genus Micromonospora. Specifically, a DNA fragment containing a neomycin-resistance gene and derived from Micromonospora sp. MK-50 (FERM BP-1613) can be mentioned. Preferred examples are a DNA fragment of about 3 kb obtained by digestion with restriction enzyme Bgl II and which is characterized in that numbers of the sites sensitive to restriction enzymes are 1 for Kpn I, 3 for Bam HI, 2 for Pst I, 2 for Sac I, 1 for Cla I, 1 for Xba I, 1 for Xho I and 1 for Bcl I; a DNA fragment of about 3 kb obtained by digestion with restriction enzyme Bam HI and which is characterized in that numbers of the sites sensitive to restriction enzymes are 1 for Pst I, 1 for Xho I, 1 for Sac I and 1 for Sph I; and a DNA fragment of about 1.5 kb obtained by digestion with restriction enzymes Bam HI/Sac I and which is characterized in that numbers of the sites sensitive to restriction enzymes are 1 for Pst I and 1 for Xho I.

A cloning vector which is capable of autonomous replication and is detectable in a microorganism of the genus Micromonospora can be constructed by digesting a plasmid capable of autonomous replication in a microorganism of the genus Micromonospora at such restriction enzyme-cleavage sites that the ability to autonomously replicate is not impaired and by introducing appropriate vector markers into the sites.

Various cloning vectors can be constructed by recombining DNA fragments obtained from plasmid pMO101 or pMO201 and harboring the function of autonomous replication in a microorganism of the genus Micromonospora with DNA fragments derived from Micromonospora sp. MK-50 and containing a neomycin-resistance gene. Examples of such cloning vectors include pMO116, pMO126, pMO133, pMO136 and pMO217.

Plasmids pMO116, pMO126, pMO133 and pMO136 are cloning vectors constructed by utilizing the ability of pMO101 to autonomously replicate in a microorganism of the genus Micromonospora and by recombining the DNA fragments of pMO101 with the DNA fragments derived from Micromonospora sp. MK-50 and containing a neomycin-resistance gene. Numbers of the sites sensitive to various restriction enzymes in the respective plasmids are shown in Tables 2 to 5. pMO116 has no sites sensitive to Eco RI and Hind III, pMO126 has no sites sensitive to Eco RI, Hind III, Bgl II, Cla I and Kpn I, pMO133 has no sites sensitive to Eco RI, Hind III and Bgl II, and pMO136 has no sites sensitive to Eco RI, Hind III, Bcl I and Cla I.

Table 2

| Cleavage Characteristics of pMO116 with Restriction Enzymes | | |
|---|---|---|
| Restriction enzyme | No. of cleavage sites | Length of DNA fragments formed by cleavage (kb) |
| Bam HI | 2 | 1.0, 5.9 |
| Bgl II | 1 | 6.9 |
| Bcl I | 1 | 6.9 |
| Pst I | 2 | 1.0, 5.9 |
| Sac I | 2 | 0.35, 6.5 |
| Xba I | 2 | 3.0, 3.9 |
| Xho I | 2 | 2.4, 4.5 |
| Cla I | 1 | 6.9 |
| Kpn I | 1 | 6.9 |
| Mlu I | 1 | 6.9 |

Table 3

| Cleavage Characteristics of pMO126 with Restriction Enzymes | | |
|---|---|---|
| Restriction enzyme | No. of cleavage sites | Length of DNA fragments formed by cleavage (kb) |
| Bam HI | 1 | 7.2 |
| Bcl I | 1 | 7.2 |
| Pst I | 2 | 1.0, 6.2 |
| Sac I | 1 | 7.2 |
| Xba I | 2 | 3.1, 4.1 |
| Xho I | 3 | 1.8, 2.4, 3.0 |
| Mlu I | 1 | 7.2 |
| Sph I | 1 | 7.2 |

Table 4

| Cleavage Characteristics of pMO133 with Restriction Enzymes | | |
|---|---|---|
| Restriction enzyme | No. of cleavage sites | Length of DNA fragments formed by cleavage (kb) |
| Bam HI | 7 | 0.64, 1.0, 1.1, 1.8, 2.5, 6.9, 8.5 |
| Bcl I | 1 | 22 |
| Pst I | 8 | 0.75, 1.35, 2.1, 2.9, 3.2, 3.3, 3.4, 5.4 |
| Sac I | 3 | 0.35, 7.1, 15 |
| Xba I | 4 | 2.7, 3.1, 5.7, 10.9 |
| Xho I | 2 | 5.0, 17 |
| Cla I | 2 | 2.4, 20 |
| Kpn I | 3 | 3.4, 6.6, 12.4 |

Table 5

| Cleavage Characteristics of pMO136 with Restriction Enzymes | | |
|---|---|---|
| Restriction enzyme | No. of cleavage sites | Length of DNA fragments formed by cleavage (kb) |
| Bam HI | 1 | 7.1 |
| Bgl II | 1 | 7.1 |
| Pst I | 2 | 1.0, 6.1 |
| Sac I | 1 | 7.1 |
| Xba I | 1 | 7.1 |
| Xho I | 2 | 3.5, 3.6 |
| Mlu I | 1 | 7.1 |

Plasmid pMO217 is a cloning vector constructed by utilizing the ability of pMO201 to autonomously replicate in a microorganism of the genus Micromonospora and by incorporating a Bgl II-cleaved DNA fragement of about 3 kb derived from Micromonospora sp. MK-50 and containing a neomycin-resistance gene. Numbers of the sites sensitive to various restriction enzymes are shown in Table 6. pMO217 has no sites sensitive to Eco RI and Hind III.

Table 6

| Cleavage Characteristics of pMO217 with Restriction Enzymes | | |
|---|---|---|
| Restriction enzyme | No. of cleavage sites | Length of DNA fragments formed by cleavage (kb) |
| Bam HI | 6 | 1.0, 1.1, 1.5, 1.7, 1.8, 5.5 |
| Bgl II | 2 | 3.0, 9.6 |
| Bcl I | 5 | 0.60, 0.75, 2.8, 4.1, 4.3 |
| Pst I | 5 | 0.90, 1.0, 1.3, 4.0, 5.4 |
| Sac I | 5 | 0.35, 1.0, 1.9, 2.2, 7.1 |
| Xba I | 1 | 12.6 |
| Xho I | 2 | 5.8, 6.8 |
| Cla I | 3 | 3.3, 4.6, 4.7 |
| Kpn I | 3 | 0.40, 5.6, 6.6 |
| Sph I | 1 | 12.6 |

Furthermore, the present invention provides shuttle vectors capable of autonomous replication in both of a microorganism of the genus Micromonospora and a microorganism of Escherichia coli. The shuttle vectors can be constructed by recombining the above-mentioned plasmid capable of autonomous replication in a microorganism of the genus Micromonospora with a plasmid capable of autonomous replication in a microorganism of Escherichia coli. The use of such shuttle vectors enables amplification of a gene derived from actinomycetes by using a microorganism of Escherichia coli instead of actinomycetes of slow growth, whereby analysis of genes and in vitro mutagenesis can be readily carried out.

The plasmids capable of autonomous replication in a microorganism of Escherichia coli include, for example, pUC19 [Gene 33, 103 (1985)], pBR322 [Gene 22, 277 (1983)], and pACYC184 [J. Bacteriol, 134, 1141 (1978)].

Recombination of a DNA fragment which harbors the function of autonomous replication in a microorganism of the genus Micromonospora with a DNA fragment capable of autonomous replication in a microorganism of Escherichia coli by utilizing the cleaved ends resulting from digestion with the same restriction enzymes gives a shuttle vector capable of autonomous replication in both microorganisms.

An example of such shuttle vectors is plasmid pMED101 constructed by recombining plasmid pMO136 capable of autonomous replication in a microorganism of the genus Micromonospora with plasmid pUC19 capable of autonomous replication in a microorganism of Escherichia coli.

pMED101 has sites sensitive to various restriction enzymes as shown in Table 7, and has no sites sensitive to Eco RI, Bcl I and Cla I.

Table 7

| Cleavage Characteristics of pMED101 with Restriction Enzymes | | |
|---|---|---|
| Restriction enzyme | No. of cleavage sites | Length of DNA fragments formed by cleavage (kb) |
| Hind III | 1 | 7.5 |
| Bam HI | 1 | 7.5 |
| Bgl II | 1 | 7.5 |
| Pst I | 2 | 0.8, 6.7 |
| Sac I | 1 | 7.5 |
| Xba I | 2 | 3.5, 4.0 |
| Xho I | 2 | 3.5, 4.0 |
| Kpn I | 1 | 7.5 |
| Mlu I | 1 | 7.5 |
| Sph I | 1 | 7.5 |

The plasmids of the present invention can autonomously replicate in a microorganism of the genus Micromonospora, and therefore, microorganisms of the genus Micromonospora, specifically the following species, can be used as host microorganisms.

Micromonospora echinospora
Micromonospora purpurea
Micromonospora sagamiensis
Micromonospora grisea
Micromonospora inyoensis
Micromonospora zionensis
Micromonospora rhodorangea
Micromonospora rosaria
Micromonospora lacustris
Micromonospora chalcea
Micromonospora olivasterospora

A mutant strain released from restriction systems is more useful as a host microorganism. Such mutant strain can be obtained according to the procedure of Lomovskaya, et al. [Microbiological Review 44 206 (1980)].

Introduction of the aforementioned plasmid into a host microorganism is carried out by the protoplast transformation method described by K.F. Chater, et al. [Current Topics in Microbiology and Immunology 96, 69-95 (1982)]. Details are shown in Examples below.

Strains respectively containing the plasmids of the present invention, pMO116, pMO126, pMO133, pMO217 and pMED101 have been deposited with the Fermentation Research Institute, Agency of Industrial Science and Technology (FERM) under the Budapest Treaty as shown in Table 8.

Table 8

| Plasmid | Strain | Accession Number (Deposition date) |
|---|---|---|
| pMO116 | Micromonospora olivasterospora MH-116 | FERM BP-1615 (12/12/'87) |
| pMO126 | Micromonospora olivasterospora MH-126 | FERM BP-1616 (12/12/'87) |
| pMO133 | Micromonospora olivasterospora MH-133 | FERM BP-1617 (12/12/'87) |
| pMO217 | Micromonospora olivasterospora MH-217 | FERM BP-1618 (12/12/'87) |
| pMED101 | Escherichia coli TD-MED101E | FERM BP-1770 (2/27/'88) |

The cloning vectors of the present invention are useful for the cloning and expression of genes in a host microorganism belonging to the genus Micromonospora. For example, when a gene responsible for the biosynthesis of an antibiotic is inserted into the cloning vector of the present invention and the recombinant vector is introduced into a microorganism belonging to the genus Micromonospora and capable of producing the antibiotic, productivity of the antibiotic by the transformant is greatly improved.

Certain embodiments of the invention are illustrated in the following examples.

Example 1

Isolation of plasmids pMO101 and pMO201

(1) Culturing

Micromonospora olivasterospora ATCC 31010 was inoculated into 4 mℓ of SK No. 2c medium [20 g of Stabirose K (Matsutani Kagaku Kogyo), 5 g of glucose, 5 g of yeast extract (Daigo Eiyo Kagaku), 5 g of peptone (Kyokuto Seiyaku Kogyo), 3 g of meat extract (Kyokuto Seiyaku Kogyo), 0.2 g of $KH_2PO_4$, 0.6 g of $MgSO_4 \cdot 7H_2O$, 1 g of $CaCO_3$ and deionized water to make up to 1 ℓ, adjusted to pH 7.6 and autoclaved at 120°C for 20 minutes] and subjected to shaking culture at 30°C for 2 to 3 days. Then, the whole culture was inoculated into 500 mℓ of SK No. 2 medium having the same composition as SK No. 2c medium except that $CaCO_3$ is not contained, and subjected to shaking culture at 30°C for 2 days.

(2) Isolation of plasmid

The culture obtained in (1) was centrifuged at 8,000 rpm at 4°C for 10 minutes to recover the cells. The cells were suspended in 40 - 50 mℓ of a lytic solution [10.3% sucrose, 25 mM tris(hydroxymethyl)-aminomethane (hereinafter abbreviated to Tris) - HCl buffer (pH 8.0), and 25 mM disodium ethylenediamine tetraacetate (EDTA)] containing 5 mg/mℓ egg white lysozyme (Seikagaku Kogyo) and incubated at 37°C for 30 minutes. To the suspension was added 30 mℓ of a 0.3N sodium hydroxide solution containing 2% sodium dodecylsulfate (Nakarai Chemicals, Ltd.), and the mixture was thoroughly stirred and allowed to stand at room temperature for 10 minutes. Then, the mixture was heated at 75°C for 10 minutes, cooled by dipping in a water bath, and admixed with a phenolchloroform mixture [the underlayer obtained by thoroughly mixing 500 mℓ of chloroform, 500 g of phenol and 800 mg of 8-hydroxyquinoline (Tokyo Kasei Kogyo), and adding thereto 200 mℓ of deionized water, followed by shaking] in a ratio of 4:3 (v/v), followed by vigorous stirring for 30 seconds. The upper layer obtained by centrifugation of the resulting mixture at 8,000 rpm at 4°C for 10 minutes was mixed with 3 M sodium acetate solution (1/10 volume of the upper layer) and then isopropyl alcohol containing 2% Triton x 100 (Nakarai Chemicals, Ltd.) (equal to volume of the upper layer) was added thereto. After being thoroughly mixed, the mixture was allowed to stand at room temperature for 30 minutes and again centrifuged at 8,000 rpm at 4°C for 10 minutes. The resulting pellets were dissolved in 20 mℓ of TE buffer [10 mM Tris-HCl buffer (pH 8.0) and 1 mM EDTA] and to the solution was added ribonuclease (Ribonuclease A, type IA, made by Sigma) which was heat-treated in advance at 80°C for 10 minutes to a final concentration of 20 μg/mℓ. After incubation at 37°C for 30 minutes, 2 mℓ of 3 M sodium acetate and 20 mℓ of isopropyl alcohol were added thereto, and the mixture was allowed to stand at room temperature for 30 minutes. The mixture was then subjected to centrifugation at 8,000 rpm at 4°C for 5 minutes to obtain pellets, which were dissolved again in TE buffer. Ethidium bromide was added to the solution to a final concentration of 0.75 mg/mℓ and 8 mℓ of the resulting solution was admixed with 8.00 g of cesium chloride. The resulting solution was centrifuged at 105,000 xg at 18°C for 40 hours. After this centrifugation, the circular DNA was detected as a specific band showing fluorescence under a ultraviolet lamp. The band was taken out with an injection needle and shaken several times together with isoamyl alcohol (Nakarai Chemicals, Ltd.) saturated with TE buffer to remove ethidium bromide, and then admixed with 3 M sodium acetate and isopropyl alcohol in a ratio of 10:1:10 (v/v/v). The mixture was allowed to stand on ice for 30 minutes. Then, pellets were recovered therefrom by centrifugation at 12,000 rpm at 4°C for 5 minutes, washed with cold 70% ethanol and dried in vacuo The dried pellets were dissolved in 0.3 mℓ of TE buffer and kept in a freeze at -20°C. Through these operations, 40 to 50 μg of pMO101 plasmid DNA was obtained.

Similarly pMO201 plasmid DNA was obtained from Micromonospora olivasterospora 3629-4 (FERM BP-1614) by the foregoing precedure.

Example 2

Construction of plasmids pNMR-1 and pNMR-10

(1) Extraction of whole DNA of Micromonospora sp. MK-50 (FERM BP-1613

Micromonospora sp. MK-50 was inoculated into 4 mℓ of SK No. 2c medium and subjected to shaking culture at 30°C for 3 days. Then, the whole culture was inoculated into 30 mℓ of SK No. 2 medium and subjected to shaking culture at 30°C for 2 days. The cells were collected by centrifugation at 12,000 rpm at 4°C for 10 minutes and washed twice with 20 mℓ of 10.3% sucrose solution. Then, the cells were suspended in 6 mℓ of TS buffer [50 mM Tris-HCl buffer (pH 8.0) containing 10.3% sucrose and 25 mM EDTA] and admixed with 50 mg of egg white lysozyme, followed by incubation at 37°C for one hour. To the mixture were added, 0.6 mℓ of proteinase K (made by Sigma, adjusted to a concentration of 2 mg/mℓ with TS buffer) and 3.6 mℓ of 3.3% sodium dodecylsulfate solution, and the mixture was incubated at 37°C for one hour for bacteriolysis. Then, the mixture was heated at 50°C for 30 minutes and cooled by dipping in a water bath.

After addition of 10 mℓ of plenol-chloroform mixture, the mixture was gently stirred for 5 minutes and centrifuged at 12,000 rpm at 4°C for 15 minutes. The upper layer was taken out and ribonuclease was added thereto to a final concentration of 20 μg/mℓ, followed by incubation at 37°C for 45 minutes. Then, a 5 M sodium chloride solution (1/10 volume of the upper layer) and polyethylene glycol 600 (Nakarai Chemicals, Ltd.) (1/4 volume of the upper layer) were added thereto, followed by gentle mixing. After being allowed to stand on ice overnight, the mixture was centrifuged at 5,000 rpm at 4°C for 5 minutes, and the resulting pellets were dissolved in 5 - 10 mℓ of TE buffer. To the solution were added 3 M sodium acetate (1/10 volume of the solution), 66 mM MgCl₂ solution (1/30 volume of the solution) and then cold 99% ethanol (2.2 times volume of the solution). The mixture was gently stirred and then centrifuged at 5,000 rpm at 4°C for 5 minutes to obtain pellets. The pellets were washed twice with cold 70% ethanol and dissolved in 4 mℓ of TE buffer, whereby a DNA solution having a concentration of about 450 μg/mℓ was obtained.

(2) Cloning of neomycin-resistance gene

The chromosomal DNA of Micromonospora sp. MK-50 obtained in (1) was partially digested with restriction enzyme Sau3 AI (Boehringer Mannheim) to make DNA fragments having a length of 4 - 6 kb as the main component. Then, 2 μg of vector pIJ702 [J. Gen. Microbiol. 129, 2703-2714 (1983)] digested with restriction enzyme Bgl II (Boehringer Mannheim) and treated with alkaline phosphatase (for molecular biology, made by Boehringer Mannheim) was added to 100 μl of a solution containing 2 μg of the DNA mentioned above, which had been agitated with a phenol-chloroform mixture to inactivate the enzyme. To the solution were added 3 M sodium acetate (1/10 volume of the solution and isopropyl alcohol (equal to volume of the solution). Then, the mixture was allowed to stand on ice for 30 minutes and centrifuged at 12,000 rpm at 4°C for 5 minutes to obtain pellets. The pellets were washed once with cold 70% ethanol and dried in vacuo.

The dried pellets were dissolved in 25 μl of ligation buffer [66 mM Tris-HCl buffer (pH 7.6) containing 6.6 mM MgCl₂, 10 mM dithiothreitol (Nakarai Chemicals, Ltd.) and 0.8 mM adenosine triphosphate (Sigma)], and one unit of T4 ligase (Boehringer Mannheim) was added to the solution. The mixture was incubated at 15°C overnight. To the reaction mixture was added, 75 μl of P-medium prepared by mixing a solution containing 10.3 g of sucrose, 25 mg of $K_2SO_4$, 203 mg of $MgCl_2 \cdot 6H_2O$ and 0.2 ml of trace element solution and made up to 90 ml with deionized water, which was sterilized in an autoclave, with 10 ml of 0.25 M TES [N-tris(hydroxymethyl)methyl-2-aminoethanesulfonic acid] buffer (pH 7.2), 0.5 ml of 5 M CaCl₂ and 1 ml of 0.5% KH₂PO₄, separately sterilized in an autoclave. The mixture was then gently admixed with 25 μl of a protoplast suspension obtained from Streptomyces lividans TK 23 strain according to a conventional procedure [Current Topics in Microbiology and Immunology 96 69-95 (1985)], and the resulting mixture was allowed to stand at room temperature for one minute. Then, 500 μl of T-medium [a mixture of 75% by volume of P-medium and 25% by volume of polyethyleneglycol 1000 (Nakarai Chemicals, Ltd.)] was added thereto, and the mixture was stirred and diluted 10 times with P-medium. The diluted mixture (100 μl) was spread on 12 plates of R5 regeneration agar plate medium prepared in the following manner: a solution (pH 7.2) containing 103 g of sucrose, 0.25 g of $K_2SO_4$, 10.12 g of $MgCl_2 \cdot 6H_2O$, 10 g of glucose, 0.1 g of casamino acids (Difco Laboratories), 5 g of yeast extract (Difco Laboratories), 2 ml of a trace element solution, 5.7 g of TES and 2.5 g of sodium hydroxide and made up to 1 l with deionized water was divided into five equal parts; each part was mixed with 4.4 g of Bactoagar (Difco Laboratories) and sterilized at 120°C for 20 minutes; to each part were added 3 ml of 20% sodium l-glutaminate, 3 ml of 2% NaNO₃, 2 ml of 0.5% KH₂PO₄ and 0.6 ml of 5M CaCl₂, followed by rapid mixing; and the resulting mixture was poured into plates in 20 ml portions, followed by drying in a clean bench for 2 hours. After culturing on R5 regeneration agar plate medium at 30°C for 15 hours, 2.5 ml of the soft agar medium [8 g of Nutrient broth (Difco Laboratories), 5 g of Bactoagar and 1 l of deionized water, sterilized at 120°C for 20 minutes] containing 0.5 mg/ml antibiotic thiopeptin (Fujisawa Pharmaceutical) was laid thereon, and culturing was continued at 30°C for 2 to 3 weeks, whereby spores were sufficiently formed. Replica plating was made on YEME medium [prepared by sterilizing in an autoclave the medium containing 3 g of yeast extract (Difco Laboratories), 5 g of Bactopeptone (Difco Laboratories), 3 g of malt extract (Difco Laboratories), 10 g of glucose and 20 g of Bactoagar in 1 l of deionized water (pH 7.2)] containing 25 μg/ml neomycin B (sulfate, made by Sigma) with velvet cloth, and culturing was further conducted at 30°C for 4 days. The colonies grown on the YEME plates were considered to contain recombinant plasmids harboring neomycin-resistance genes, and the plasmids were recovered in the same manner as in Example 1. It was found as the result of digestion with various restriction enzymes and analysis by agarose gel electrophoresis that two different genes expressing neomycin resistance were inserted in the obtained two plasmids, pNMR-1 and pNMR-10.

Example 3

Construction of cloning vectors pMO116 and pMO133

pMO101 (3.8 μg) obtained in Example 1 was added to 30 μl of M buffer [Manual for Genetic Engineering (Cold Spring Harbor Laboratory), p.228 (1980)] containing 8 units of restriction enzyme Bcl I (Boehringer Mannheim) and incubated at 50°C for 2 hours. To the mixture were successively added 60 μl of deionized water, 10 μl of 1 M Tris-HCl buffer (pH 8.6) and 1.5 units of alkaline phosphatase (for molecular biology), and the mixture was incubated at 37°C for 45 minutes. Then, 100 μl of a phenol-chloroform mixture was added thereto, and the mixture was vigorously stirred for 30 seconds and centrifuged at 12,000 rpm at 18°C for 5 minutes. The resultant upper layer was admixed with about 2 μg of Bgl II-digested DNA fragment of 3 kb of pNMR-1 obtained in Example 2 and then with 3 M sodium acetate (1/10 volume of the upper layer) and isopropyl alcohol (equal to volume of the upper layer), and the mixture was allowed to stand on ice for 30 minutes. The pellets obtained by centrifugation at 12,000 rpm at 4°C for 5 minutes were washed with cold 70% ethanol and dried in vacuo To the dried pellets were added, 20 μl of ligation buffer and one unit of T4 ligase, and the mixture was allowed to stand at 15°C overnight. Then, 80 μl of P-medium was added to the resulting reaction mixture, and the protoplast suspension of Micromonospora olivasterospora ATCC 21819 prepared by the same method as used in Example 2 (2) for the preparation of protoplasts of Streptomyces lividans TK23 strain was quickly suspended therein. The mixture was allowed to stand at room temperature for one minute and gently mixed with 500 μl of T-medium. Then, the mixture was diluted 10 times with P-medium, and 100 μl portions of the diluted mixture were spread on 24 plates of regeneration agar plate medium RM-1 prepared in the following manner: a solution (pH 8.0) containing 100 g of sucrose, 10 g of Stabirose K (Matsutani Kagaku Kogyo), 50 mg of $KH_2PO_4$, 5.1 g of $MgCl_2 \cdot 6H_2O$, 3.7 g of $CaCl_2 \cdot 2H_2O$, 0.5 g of peptone (Kyokuto Seiyaku), 1.0 g of yeast extract (Daigo Eiyo Kagaku), 50 mg of $FeSO_4 \cdot 7H_2O$, 5.6 g of TES and 20 g of Bactoagar (Difco Laboratories) and made up to 1 ℓ with deionized water was sterilized at 120°C for 20 minutes; and 25 mℓ portions of the solution were poured into the plates and solidified. After culturing on RM-1 medium at 30°C for 4 days, 2.5 mℓ of the soft agar medium containing 0.4 mg/mℓ neomycin B was laid thereon and the plates were subjected to further culturing at 30°C for 14 days. The regenerated colonies which were neomycin-resistant were transferred into ATCC S-5 medium [a solution (pH 7.2) containing 10 g of Stabirose K, 1 g of yeast extract (Daigo Eiyo Kagaku), 1 g of meat extract (Kyokuto Seiyaku), 2 g of Bactotriptose (Difco Laboratories), 100 mg of $FeSO_4 \cdot 2H_2O$ and 20 g of Bactoagar and made up to 1 ℓ with deionized water, which was sterilized at 120°C for 20 minutes] containing neomycin B at a concentration of 2.5 μg/mℓ, and cultured at 30°C for 5 days. Plasmids carried by the microorganisms grown on the plates were recovered in the same manner as in Example 1, whereby two plasmids, pMO116 and pMO133, were obtained.

Example 4

Construction of pMO126

Restriction enzymes Bam HI and Bgl II (4 units each) were added to 30 μℓ of M buffer containing 1 μg of pMO116, followed by incubation at 37°C for 2.5 hours. To the mixture were added 60 μℓ of deionized water, 10 μℓ of 1 M Tris-HCl buffer (pH 8.6) and one unit of alkaline phosphatase (for molecular biology), and the mixture was incubated at 37°C for 45 minutes. Then, 100 μℓ of a phenol-chloroform mixture was added thereto, and the mixture was vigorously stirred and centrifuged at 12,000 rpm at 15°C for 5 minutes. The resulting supernatant was admixed with 0.5 μg of Bam HI-digested DNA fragment of 3 kb obtained from pNMR-10, 3 M sodium acetate (1/10 volume of the supernatant) and isopropyl alcohol (equal to volume of the supernatant), and the mixture was allowed to stand on ice for 30 minutes. Subsequent steps were carried out in the same manner as in Example 3, and a plasmid was isolated from the regenerated strain of Micromonospora olivasterospora ATCC 21819 which was neomycin-resistant, whereby plasmid pMO126 was obtained.

Example 5

Construction of pMO136

Restriction enzymes Bam HI and Sac I (4 units each, made by Boehringer Mannheim) were added to 30 μℓ of M buffer containing 1 μg of pMO116, followed by incubation at 37°C for 2.5 hours. To the mixture were added 60 μℓ of deionized water, 10 μℓ of 1 M Tris-HCl buffer (pH 8.6) and one unit of alkaline phosphatase (for molecular biology), and the mixture was incubated at 37°C for 45 minutes. Then, 100 μℓ of a phenol-chloroform mixture was added thereto, and the mixture was vigorously shaken for 30 seconds and centrifuged at 12,000 rpm at 15°C for 5 minutes. The resulting supernatant was admixed with about 0.5 μg of Bam HI/Sac I-digested DNA fragment of 1.5 kb obtained from pNMR-10, 3 M sodium acetate (1/10 volume of the supernatant) and isopropyl alcohol (equal to volume of the supernatant), and the mixture was allowed to stand on ice for 30 minutes. Subsequent steps were carried out in the same manner as in Example 3, and a plasmid was isolated from the regenerated strain of Micromonospora olivasterospora ATCC 21819 which was neomycin-resistant, whereby plasmid pMO136 was obtained.

Example 6

Construction of pMO217

About 2 μg of pMO201 was added to 30 μl of M buffer containing 4 units of restriction enzyme Bgl II (Boehringer Mannheim), followed by incubation at 37°C for 2.5 hours. To the mixtrue were added 60 μl of

deionized water, 10 μl of 1 M Tris-HCl buffer (pH 8.6) and one unit of alkaline phophatase (for molecular biology), and the mixture was incubated at 37°C for 45 minutes. Then, 100 μl of a phenol-chloroform mixture was added thereto, and the mixture was vigorously stirred for 30 seconds and centrifuged at 12,000 rpm at 18°C for 5 minutes. The resulting supernatant was admixed with 0.5 μg of Bgl II-digested DNA fragment of 3 kb derived from pNMR-1, 3 M sodium acetate (1/10 volume of the supernatant) and isopropyl alcohol (equal to volume of the supernatant), and the mixture was allowed to stand on ice for 30 minutes. Subsequent steps were carried out in the same manner as in Example 3, and a plasmid was isolated from the regenerated strain of Micromonospora olivasterospora ATCC 21819 which was neomycin-resistant, whereby plasmid pMO217 was obtained.

Example 7

Construction of pMED101

(1) Digestion of pMO136 with restriction enzymes

pMO136 (2 μg) obtained in Example 5 was added to 30 μl of M buffer containing 2 units of restriction enzyme Bgl II and 2 units of restriction enzyme Sac I (both restriction enzymes were made by Boehringer Mannheim), followed by incubation at 37°C for 2 hours. The mixture was made up to 100 μl with deionized water, and an equal amount of a phenol-chloroform mixture was added thereto. The mixture was vigorously stirred for 30 seconds and then centrifuged at 12,000 rpm at 18°C for 5 minutes. The resulting supernatant was admixed with 3 M sodium acetate (1/10 volume of the supernatant) and isopropyl alcohol (equal to volume of the supernatant), and the mixture was allowed to stand on ice for 30 minutes. The pellets obtained by centrifugation of the mixture at 12,000 rpm at 4°C for 5 minutes were washed with cold 70% ethanol and dried in vacuo.

(2) Conversion of Aat II site of pUC19 to Bgl II site

Plasmid pUC19 (3.5 μg) was added to 50 μl of M buffer containing 5 units of restriction enzyme Aat II (Toyobo), followed by incubation at 37°C for 2 hours. The mixture was made up to 100 μl with deionized water, and an equal amount of a phenol-chloroform mixture was added thereto. The mixture was vigorously stirred and then centrifuged at 12,000 rpm at 18°C for 5 minutes. The resulting upper layer was admixed with 3 M sodium acetate (1/10 volume of the upper layer) and isopropyl alcohol (equal to volume of the upper layer). The mixture was allowed to stand on ice for 30 minutes and centrifuged at 12,000 rpm at 4°C for 5 minutes. The resulting pellets were washed with cold 70% ethanol and dried in vacuo. The dried pellets were redissolved in 20 μl of the buffer for T4 polymerase [a solution containing 0.033 M Tris-HCl buffer (pH 7.9), 0.066 M potassium acetate, 0.01 M magnesium acetate, 0.5 mM dithiothreitol, 0.1 mM dATP (deoxyadenosine triphosphate, made by Sigma), 0.1 mM dGTP (deoxyguanosine triphosphate, made by Sigma), 0.1 mM dCTP (deoxycytidine triphosphate, made by Sigma) and 0.1 mM dTTP (deoxythimidine triphosphate, made by Sigma)], and 5 units of T4 DNA polymerase (Takara Shuzo) was added to the solution. The mixture was subjected to incubation at 37°C for 5 minutes and then made up to 100 μl with deionized water. After addition of an equal amount of a phenol-chloroform mixture, the mixture was vigorously stirred and centrifuged at 12,000 rpm at 18°C for 5 minutes. The resulting upper layer was admixed with 3 M sodium acetate (1/10 volume of the upper layer) and isopropyl alcohol (equal to volume of the upper layer). The mixture was allowed to stand on ice for 30 minutes and then centrifuged at 12,000 rpm at 4°C for 5 minutes. The resulting pellets were washed with cold 70% ethanol and dried in vacuo

Then, the dried pellets were dissolved in ligation buffer together with 1.2 μg of Bgl II linker d (pC-A-G-A-T-C-T-G, made by Takara Shuzo), and one unit of T4 ligase (Boehringer Mannheim) was added to the solution. The mixture was subjected to incubation at 15°C overnight and then made up to 100 μl with deionized water. After addition of an equal amount of a phenol-chloroform mixture, the mixture was vigorously stirred and centrifuged at 12,000 rpm at 18°C for 5 minutes. The resulting upper layer was admixed with 3 M sodium acetate (1/10 volume of the upper layer) and isopropyl alcohol (equal to volume of the upper layer). The mixture was allowed to stand on ice for 30 minutes and then centrifuged at 12,000 rpm at 4°C for 5 minutes. The resulting pellets were washed with cold 70% ethanol and dried in vacuo.

13

(3) Preparation of Bgl II-Sac I fragment derived from pUC19

The DNA fragment prepared in (2) above, wherein the Aat II site of pUC19 was converted to Bgl II site, was added to 30 μℓ of M buffer containing 20 units of restriction enzyme Bgl II and 4 units of restriction enzyme Sac I (both restriction enzymes were made by Boehringer Mannheim), followed by incubation at 37°C for 2 hours. The mixture was made up to 100 μℓ with deionized water, and 10 μℓ of 1 M Tris-HCl buffer (pH 8.6) and 1.5 units of alkaline phosphatase (for molecular biology) were successively added thereto. The mixture was subjected to incubation at 37°C for one hour. After agarose gel electrophoresis, the desired DNA fragment digested with Bgl II and Sac I was cut out of the agarose gel and electrically eluted in a dialysis tube. To the eluate was added a phenol-chloroform mixture (equal to volume of the eluate), and the mixture was vigorously stirred for 30 seconds and centrifuged at 12,000 rpm at 18°C for 5 minutes. The resulting upper layer was admixed with 3 M sodium acetate (1/10 volume of the upper layer) and isopropyl alcohol (equal to volume of the upper layer). The mixture was allowed to stand on ice for 30 minutes and centrifuged at 12,000 rpm at 4°C for 5 minutes. The resulting pellets were washed with cold 70% ethanol and dried in vacuo.

(4) Ligation of pMO136 digested with Bgl II-Sac I with the Bgl II-Sac I DNA fragment derived from pUC19

The Bgl II-Sac I DNA fragment (about 5.3 kb) derived from pMO136 and obtained in (1) and the Bgl II-Sac I fragment (about 2.2 kb) derived from pUC19 and obtained in (3) were dissolved in 30 μℓ of ligation buffer and one unit of T4 ligase (Boehringer Mannheim) was added to the solution. The mixture was incubated at 15°C overnight. To the mixture were added 3 M sodium acetate (1/10 volume of the mixture) and isopropyl alcohol (equal to volume of the mixture). The mixture was allowed to stand on ice for 30 minutes and then centrifuged at 12,000 rpm at 4°C for 5 minutes. The resulting pellets were washed with cold 70% ethanol, dried in vacuo and suspended in 10 μℓ of TE buffer.

(5) Transformation of Escherichia coli K-12:HB101 and preparation of pMED101

Competent cells of Escherichia coli K-12:HB101 strain (100 μℓ) prepared according to a conventional procedure [Bolivar, et al.: Gene 2: 75-93 (1977)] was mixed with the in vitro recombinant DNA (about 1.0 μg) obtained in (4), and the mixture was subjected to incubation on ice for 20 minutes and then at 37°C for 5 minutes. To the mixture was added 1.0 mℓ of L-medium [a solution (pH 7.0) containing 10 g of Bactotryptone (Difco Laboratories), 5 g of yeast extract (Difco Laboratories) and 5 g of NaCl and made up to 1 ℓ with deionized water, which was sterilized at 120°C for 20 minutes], followed by incubation at 37°C for one hour. A part of the mixture was appropriately diluted and spread on L-medium agar plate [L-medium containing 1.5% Bactoagar (Difco Laboratories)] containing 50 μg/mℓ ampicillin. Colonies which grew on the plates by acquiring ampicillin-resistance were selected and further cultured in L-medium containing 50 μg/mℓ ampicillin at 37°C overnight. Plasmids carried by the cells were isolated in the same manner as in Example 1, whereby pMED101 was obtained.

Example 8

Preparation of restriction enzyme cleavage maps for various plasmids

Plasmids pMO101, pMO116, pMO126, pMO133, pMO136, pMO217 and pMED101 obtained in Examples 1 to 7 were respectively analyzed by digestion with various restriction enzymes and agarose gel electrophoresis to prepare restriction enzyme cleavage maps shown in Figs. 1 to 7.
The neomycin-resistance genes derived from Micromonospora sp. MK-50 and cloned on pNMR-1 and pNMR-10 were likewise analyzed to prepare restriction enzyme cleavage maps for the DNA fragments containing the neomycin-resistance genes (see Figs. 8 and 9).

Example 9

14

Transformation of Micromonospora olivasterospora with pMO116, pMO133 and pMO217

Micromonospora olivasterospora ATCC 21819 strain was transformed with 1 μg each of pMO116, pMO133 and pMO217 prepared above, individually in the same manner as in Example 3. Numbers of the transformed cells obtained with pMO116, pMO133 and pMO217 were 5.0 x 10⁵, 1.2 x 10⁷, and 1.1 x 10⁷, respectively, and thus very high transformation efficiencies were obtained.

Example 10

Transformation of Micromonospora olivasterospora with pMED101

To about 1.0 μg of pMED101 obtained in Example 7 was added P-medium to make up to 100 μℓ, and 50 μℓ of a protoplast suspension of Micromonospora olivasterospora ATCC 21819 was quickly added thereto. The mixture was allowed to stand at room temperature for one minute and then 600 μℓ of T-medium was added with gentle mixing. The mixture was diluted 10 times with P-medium, and 100 μℓ portions of the diluted mixture were spread on 24 plates of regeneration agar plate medium RM-1. By culturing on the agar medium, the protoplasts were regenerated as normal hypha cells. After culturing at 30°C for 4 days, 2.5 mℓ of the soft agar medium containing 0.4 mg/mℓ neomycin B (Sigma) was laid on the plates and solidified. Then, the plates were subjected to further culturing at 30°C for 3 weeks.

The transformants grown on the plates by acquiring neomycin resistnace were cultured in SK No. 2 medium and the plasmids carried by the transformants were investigated in the same manner as in Example 1. It was found that all of these transformants contained pMED101. In this test, 132 transformed cells were obtained with 1 μg of pMED101.

Example 11

Cloning and expression of a gene responsible for the production of the antibiotic fortimicin A

(1) Incorporation of chromosomal DNA derived from Micromonospora olivasterospora ATCC 21819 into pMO116

The whole chromosomal DNA of Micromonospora olivasterospora ATCC 21819 which produces fortimicin A was extracted from the strain by the method described in Example 2 (1). The chromosomal DNA was partially digested with restriction enzyme Sau3 Al (Boehringer Mannheim) to make DNA fragments having a length of 6 - 8 kb by sucrose density gradient centrifugation. That is, a density gradient of 40 -10% sucrose solution was prepared in six sterilized Hitachi 40 PA tubes (Nissei Sangyo) by gently packing equal amounts of 20 mM Tris-HCl buffer solutions (pH 8.0) containing 1 M NaCl, 5 mM EDTA and, respectively, 40, 30, 20 and 10% sucrose to make up to 38 mℓ. Then, the solution containing 200 μg of the DNA fragments mentioned above was heated at 65°C for 15 minutes and cooled. The solution was gently put on the sucrose solution in the tube and the tube was set in Hitachi SRP 28 Rotor (Nissei Sangyo) and subjected to centrifugation at 26,000 rpm at 2°C for 24 hours. The supernatant in each tube was gently put into 30 Eppendorf tubes and fractions containing DNA fragments of 6 - 8 kb were collected by agarose gel electrophoresis. To the fractions was added TE buffer (4 times volume of the fractions), and then twice as much cold ethanol as the mixture by volume was added. The precipitated DNA (total amount: 82 μg) was recovered by centrifugation at 12,000 rpm for 5 minutes.

To 7.5 μg of the DNA thus obtained was added 2 μg of pMO116 which had been in advance completely digested with restriction enzyme Bgl II (Boehringer Mannheim) and treated with alkaline phosphatase (for molecular biology, made by Boehringer Mannheim). The mixture was dissolved in 450 mℓ of ligation buffer. The solution was allowed to stand on ice for 30 minutes, and 3 units of T4 ligase (Boehringer Mannheim) was added, followed by incubation at 15°C for 2 hours to obtain a ligation product.

(2) Transformation of fortimicin A-non-producing mutant and cloning of fortimicin A biosynthesis gene

The ligation product obtained in (1) was subjected to precipitation with isopropyl alcohol, and the precipitate was dissolved in 20 μℓ of TE buffer, followed by addition of 80 μℓ of P-medium.

To the mixture was added 50 μl of the protoplast suspension obtained from fortimicin A-non-producing mutants Micromonospora olivasterospora AN 58-1 and AN 25-2 (about 2 x 10$^9$ protoplasts/mℓ) by the method described in Example 9.

After gentle mixing, the mixture was allowed to stand at room temperature for one minute, and 600 μℓ to T-medium was added thereto. The mixture was diluted 10 times with P-medium, and 100 μℓ portions of the diluted solution were spread on 80 plates for regeneration, followed by incubation at 30°C for 6 days. Then, 2.5 mℓ of the soft agar medium having the same composition as that used in Example 2 (2) and containing 40 μg/mℓ (final concentration) neomycin B was put on the plate, followed by incubation at 30°C for 3 weeks.

The thus obtained neomycin-resistant transformants were inoculated on plates of an agar medium for the production of fortimicin A containing 2.5 μg/mℓ neomycin B, which was prepared in the following manner: a solution (pH 7.1) containing 1 g of maltose, 1 g of yeast extract (Difco Laboratories), 1 g of peptone (Kyokuto Seiyaku), 0.6 g of meat extract (Kyokuto Seiyaku), 4.55 g of TES, 40 mg of KH$_2$PO$_4$, 120 mg of MgSO$_4$·7H$_2$O and 20 g of Bactoagar (Difco Laboratories) and made up to 1 ℓ with deionized water was sterilized at 120°C for 20 minutes; and 20 mℓ portions of the solution were poured into the plates and solidified. Culturing was carried out at 30°C for 6 days.

To each plate was added 15 mℓ of a soft agar medium (pH 7.5) containing 10$^8$ m/mℓ Escherichia coli cells, which was prepared in the following manner: a solution containing 2.5 g of yeast extract (Difco Laboratories), 3 g of beef extract (Difco Laboratories), 6 g of Bacto-triptone (Difco Laboratories), 1 g of glucose, 12.9 g of Tris and 10 g of Bactoagar (Difco Laboratories) and made up to 1 ℓ with deionized water was sterilized at 120°C for 20 minutes; and Escherichia coli was inoculated into the solution kept at 42°C.

After solidification, the plates were subjected to incubation at 37°C for 15 hours.

As the result, it was found that one neomycin-resistant transformant derived from AN 58-1 strain and one neomycin-resistant transformant derived from AN 25-2 strain restored their productivity of fortimicin A.

Plasmids were recovered from these transformants by the method described in Example 1 and named pAN 25-10 and pAN 58-10. Restriction enzyme cleavage maps for the plasmids were made and illustrated in Fig. 10, A and B. On the DNA fragments inserted into these plasmids, there exist genes coding for the enzymes relating to the fortimicin biosynthesis, i.e. the enzyme at step 3 wherein the intermediate compound Fu-10 is produced from scyllo-inosamine and glucosamine and the enzyme at step 11 wherein the intermediate compound KR is produced from the intermediate compound KH.

(3) Improvement of productivity of fortimicin A by using recombinant plasmids

Plasmids pAN 25-10 and pAN 58-10 were incorporated into the fortimicin-producing strain G-2 [Journal of Antibiotics 37, 1670 (1984)] derived from Micromonospora olivasterospora ATCC 21819 by the method described in Example 3.

G-2 strain and the thus obtained transformants were cultured by the following method. That is, the first seed culture was carried out using 4 mℓ of SK No. 2 medium containing 0.25 μg/mℓ neomycin B at 30°C for 3 days with shaking. For the second seed culture, 1 mℓ of the first seed culture was inoculated into 10 mℓ of the same medium as the first seed medium and cultured with shaking at 30°C for 24 hours. Then, 3 mℓ of the culture was inoculated into 30 mℓ of a liquid medium for the production of fortimicin A (pH 7.0) containing 7.5 μg of neomycin B in a 300-mℓ flask. Composition of the medium are as follows: 40 g/ℓ maltose, 5 g/ℓ glucose, 10 g/ℓ peptone (Kyokuto Seiyaku), 15 g/ℓ soybean meal (Kyokuto Seiyaku), 1 g/ℓ KH$_2$PO$_4$, 3 g/ℓ K$_2$HPO$_4$, 3 g/ℓ MgSO$_4$·7H$_2$O, 2 g/ℓ NaCl, 10 mg/ℓ FeSO$_4$·7H$_2$O, 10 mg/ℓ NiCl$_2$·6H$_2$O, 10 mg/ℓ ZnSO$_4$·7H$_2$O, 10 mg/ℓ CuSO$_4$·5H$_2$O, 50 mg/ℓ MnSO$_4$·4-5H$_2$O, 1 mg/ℓ COCl$_2$, 10 mg/ℓ D-calcium pantothenate and 50 g/ℓ dry yeast (Oriental Yeast Industry). In preparing the medium, a solution of sugar components and a solution of the other components were separately sterilized at 120°C for 20 minutes and combined. Culturing was carried out on a rotary shaker at 30°C for 7 days. Then, 2 mℓ of the culture was recovered and 0.3 mℓ of 2N H$_2$SO$_4$ was added. The mixture was shaken at 28°C for 30 minutes and then subjected to centrifugation at room temperature at 3,000 rpm for 20 minutes. Precoated plate No. 6721 (Merck) was spotted with 2 μℓ of the supernatant and development was carried out using the lower layer of chloroform : methanol : aqueous ammonia (1:1:1). The plate was treated in an oven to thoroughly remove ammonia and the amount of fortimicin A was determined by the fluorescence colorimetric method [Van Hoof, et al.; Analytical Chemistry 46, 286 (1974)]. The results are shown in Table 9.

16

Table 9

| Strain | Amount of fortimicin A produced ($\mu$g/m$\ell$) |
|---|---|
| G-2 | 210 |
| Strain carrying pAN 25-10 | 262 |
| Strain carrying pAN 58-10 | 270 |

The results show that the insertion of the recombinant vectors into the host cells increases the productivity of fortimicin A and that the plasmids of the present invention are useful for the cloning and expression of genes in a microorganism of the genus Micromonospora.

## Claims

1. Plasmid pMO101 which is derived from a microorganism of the genus Micromonospora and which has a molecular weight of about 25 megadaltons, wherein the number of the sites sensitive to restriction enzymes is 1 for Hind III, 5 for Bam HI, 3 for Bcl I, 9 for Pst I, 1 for Sac I, 3 for Xba I, 1 for Xho and 6 for Kpn I.

2. A recombinant cloning vector which is a recombinant plasmid prepared by incorporating a DNA fragment containing a gene capable of expressing a genetic character in a microorganism of the genus Micromonospora into a plasmid capable of autonomous replication in a microorganism of the genus Micromonospora, the cloning vector being capable of autonomous replication in a microorganism of the genus Micromonospora and being detectable in a microorganism cell on the basis of expression of the incorporated gene.

3. The recombinant cloning vector according to Claim 2, wherein the DNA fragment contains a drug-resistance gene.

4. The recombinant cloning vector according to Claim 2 or 3, wherein the DNA fragment contains a neomycin-resistance gene.

5. The recombinant cloning vector according to any one of Claims 2 to 4, wherein the DNA fragment is derived from Micromonospora sp. MK-50 (FERM BP-1613).

6. The recombinant cloning vector according to Claim 5, wherein the DNA fragment is a DNA fragment of about 3 kilobases obtained by digestion with the restriction enzyme Bgl II and which is characterized in that the number of the sites sensitive to restriction enzymes is 1 for Kpn I, 3 for Bam HI, 2 for Pst I, 2 for Sac I, 1 for Cla I, 1 for Xba I, 1 for Xho I and 1 for Bcl I; a DNA fragment of about 3 kilobases obtained by digestion with the restriction enzyme Bam HI and which is characterized in that the number of the sites sensitive to restriction enzymes is 1 for Pst I, 1 for Xho I, 1 for Sac I and 1 for Sph I; or a DNA fragment of about 1.5 kilobases obtained by digestion with the restriction enzymes Bam HI/Sac I and which is characterized in that the number of the sites sensitive to restriction enzymes is 1 for Pst I and 1 for Xho I.

7. The recombinant cloning vector according to any one of Claims 2 to 6 which is a plasmid selected from the group consisting of pMO116, pMO126, pMO133, pMO136 and pMO217.

8. The recombinant cloning vector according to any one of Claims 2 to 6 which is a plasmid capable of autonomous replication in both of a microorganism of the genus Micromonospora and a microorganism of Escherichia coli.

9. The recombinant cloning vector according to Claim 8 which is pMED101.

Fig. 1

Fig. 2

Fig. 3

pMO133

(22.4Kb)

EP 0 344 767 A1

Fig. 5

pMO136
(7.1Kb)

Fig. 4

pMO126
(7.2Kb)

Fig. 6

Fig. 7

Fig. 8

Fig. 9

Fig. 10

A                                   B

BglII/Sau3A                    BglII
KpnI                           SacI
XhoI

BglII                          MluI

PstI                           KpnI
                               BamHI
                               SacI
SacI                           KpnI
MluI
KpnI
BglII
BamHI                          SacI
PstI
BclI                           XhoI
PstI

SacI                           XhoI
PstI
BglII                          SacI
                               MluI
BglII              6.9          BglII/Sau3A

                               Kb

KpnI

BclI

XhoI
PstI

SacI

                               1Kb

11.9   BglII/Sau3A

Kb

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 064 350 (TAKEDA) --- | | C 12 N 15/00 |
| A | CHEMICAL ABSTRACTS, vol. 103, no. 3, 22nd July 1985, page 157, abstract no. 17787e, Columbus, Ohio, US; & JP-A-60 47 684 (TANABE SEIYAKU CO.) 15-03-1985 --- | | |
| A | CHEMICAL ABSTRACTS, vol. 105, no. 11, 15th September 1986, page 157, abstract no. 92152p, Columbus, Ohio, US; T. OSHIDA et al.: "Isolation and characterization of plasmids from Micromonospora zionensis and Micromonospora rosaria", & PLASMID 1986, 16(1), 74-6 --- | | |
| D,A | CHEMICAL ABSTRACTS, vol. 108, no. 25, 20th June 1988, page 309, abstract no. 218857e, Columbus, Ohio, US; P. MATSUSHIMA et al.: "Genetic transformation of Micromonospora rosaria by the Streptomyces plasmid pIJ702", & J. ANTIBIOT. 1988, 41(4), 583-5 ----- | | **TECHNICAL FIELDS SEARCHED (Int. Cl.4)** C 12 N |

The present search report has been drawn up for all claims

| Place of search | Date of completion of the search | Examiner |
|---|---|---|
| THE HAGUE | 12-07-1989 | PULAZZINI A.F.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO FORM 1503 03.82 (P0401)